Europäisches Patentamt

European Patent Office

Office européen des brevets

(11) Publication number: **0 203 443 B1**

## EUROPEAN PATENT SPECIFICATION

(45) Date of publication of patent specification: **04.12.91**

(51) Int. Cl.⁵: **G01N 33/53**, //G01N33/569, G01N33/74

(21) Application number: **86106474.9**

(22) Date of filing: **13.05.86**

(54) **Test kit for the carrying out of chemical analyses.**

(30) Priority: **14.05.85 FI 851915**

(43) Date of publication of application:
**03.12.86 Bulletin 86/49**

(45) Publication of the grant of the patent:
**04.12.91 Bulletin 91/49**

(84) Designated Contracting States:
**AT BE CH DE FR GB IT LI NL SE**

(56) References cited:
| EP-A- 0 113 075 | EP-A- 0 125 118 |
| EP-A- 0 171 150 | WO-A-83/03677 |
| FR-A- 2 516 654 | FR-A- 2 573 872 |

(73) Proprietor: **Orion Corporation Ltd**
**Saunatontuntie 1**
**SF-02100 Espoo(FI)**

(72) Inventor: **Niskanen, Aimo Juhani**
**Peipontie 20**
**SF-02210 Espoo(FI)**
Inventor: **Kahma, Kauko Ilmari**
**Niittykuja 2 B 42**
**SF-02200 Espoo(FI)**

(74) Representative: **Vossius & Partner**
**Siebertstrasse 4 P.O. Box 86 07 67**
**W-8000 München 86(DE)**

## Description

The invention comprises a test kit for the carrying out of chemical, particularly immunochemical analyses in environments other than fully-equipped scientific laboratories. With the aid of the invention a chemical analysis can be carried out and the result obtained without any additional equipment at any place whatsover.

Immunochemical analyses, eg. Pregnancy tests, can currently be performed as follows: a urine sample is pipetted to a test tube, an analysis stick is added to the same tube and incubated for a certain length of time before the stick is removed from the tube and rinsed. The stick is then transferred to a second tube, containing reagent pipetted from a storage bottle, and incubated for a given time, rinsed again and added to a third test tube to which reagent has been pipetted from a second bottle. The stick is incubated in this third tube for a given time, after which the test result is read as a colour indication either from the liquid in the tube or directly from the surface of the stick. A method of this type has been described in US-A-4 496 654 and in EP-A-0125118.4. A disadvantage of methods of the type described above is that the reagents must be pipetted into separate test tubes, which must be supported in a special rack. It is therefore possible that the order of pipetting may be incorrect in practice. In addition, rinsing must in many cases be performed under a tap, which restricts the performance of the assay to the vicinity of a tap.

EP-A 0 171 150 (a document according to Art. 54(3) EPC) discloses a test kit for use in multistep immunological analyses of one or more analytes which comprises a solid support having bound thereto a plurality of receptors and an apparatus consisting of a plurality of compartments containing reagents.

EP-A 0 125 118 discloses a method for colorimetric immunoassays wherein an antibody-coated dipstick is used.

EP-A 0 113 075 discloses a field immunoassay system comprising a dipstick probe or carrier coated with a specific monoclonal antibody to detect an antigen in body substances or fluids, and a plurality of containers containing the reagents for the different steps.

FR-A 2 156 654 discloses an apparatus for chemical and immunochemical analyses comprising a modified microtiter plate and insertion elements which may be antibody-coated and which fit into corresponding rows of wells in the microtiter plate.

We have developed a test kit with which the disadvantages cited above can be overcome.

The present invention provides a test kit for chemical and immunochemical analyses comprising

   a) an apparatus having one or more wells (7) made of an impervious material, being covered by an impervious foil and containing all reagents including rinsing solutions necessary for a given chemical or immunochemical reaction in liquid state; and

   b) a testing stick having a sharp point (1) for piercing the impervious foil covering the wells of said apparatus.

The apparatus includes a base made of plastic or some other material impervious to water, in which has been moulded a sufficient number of wells for all the reagents required in the analysis. The wells are filled with the reagents necessary for the carrying out of the reaction, in the appropriate order. The wells are covered with aluminium foil or some other impervious material, on which the positions of the wells and other instructions necessary for the carrying out of the analysis can be written. When the assay is carried out a stick of plastic or some other material is used to pierce the aluminium foil and the stick is left standing in the well containing reagents. When the requisite lenght of time has passed, the stick is lifted out of the well and transferred to the next well by piercing its aluminium foil covering. This procedure is continued until all the reagent wells have been pierced.

The invention is illustrated with the aid of the appended figures, of which Fig. 1 represents the test base seen from the side, Fig. 2 the test base seen from above, Fig. 3 the sampling spoon to be used with the apparatus, seen from above, Fig.4 represents the sampling spoon as seen from the side and Fig.5 represents the test stick as seen from above.

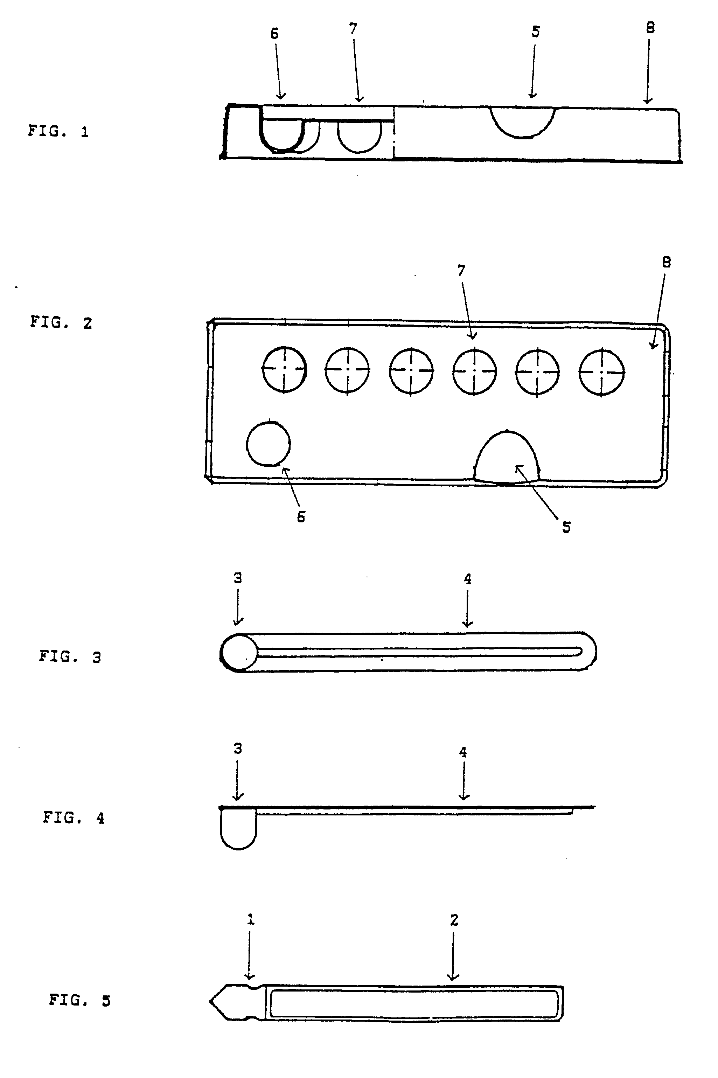

The test base as seen from the side is represented in Figure 1. The reference signs in this figure are as follows: 5, thumbhold; 6, rest for the sampling spoon; 7, reagents wells; 8, the level of the base. The test base as seen from above is represented in Figure 2. The reference sign 5 represents the thumbhold, 6 represents the rest for the sampling spoon, 7 reagent wells and 8 the level of the base.

The sampling spoon as seen from above is presented in Figure 3. The reference sign 3 represents the sample dish and 4 the stem of the sampling spoon.

The sampling spoon as seen from the side is presented in Figure 4. The reference sign 3 represents the sample dish and 4 the stem of the sampling spoon.

The testing stick as seen from above is presented in Figure 5. The reference sign 1 represents the reactive end of the stick and 2 represents the stem.

In the following we shall describe in more detail the apparatus in accordance with this invention, with examples illustrating the use of the apparatus in the carrying out of an immunochemical analysis.

Example 1

Assay of Streptococcus mutans bacteria from saliva samples.

A testing stick coated with antibodies produced against the bacterium Streptococcus mutans is incubated in a sampling spoon containing a sample of saliva for 30 minutes at room temperature. The stick is then removed from the sample and its sharp end is pushed through the aluminium foil covering the first well of the test base, as a result of which the stick is immersed in the rinsing solution contained in the first well. The stick is then removed from the first well and pushed through the aluminium foil covering the second well. This well contains antibodies produced against the bacterium S. mutans and to which molecules of the enzyme alkaline phosphatase have been chemically attached. The testing stick is kept in this well for 30 minutes, removed and used to pierce the aluminium foil covering the third well of the test base. This well contains another rinsing solution. The stick is removed from the well immediately and used to pierce the aluminium foil covering the fourth well, containing chromogenic substrate for the alkaline phosphatase enzyme. The alkaline phosphatase enzyme removes the phosphate group from the chromogenic substrate molecule, after which the molecules thus formed react with each other or with other molecules to produce a coloured substance, insoluble in water, which precipitates on the surface of the test stick. The stick is held in this well for 10 minutes, removed and examined visually for immediate estimation of the test result.

Example 2

Assay of lutropin from urine.

A testing stick coated with antibodies produced against the β-subunit of lutropin is incubated in a sampling spoon containing a sample of urine for 10 minutes at room temperature. The stick is then removed from the spoon and its sharp end is used to pierce the aluminium foil covering the first well of the test base, as a result of which the stick is immersed in the rinsing solution contained in the first well. After this the stick is removed and pushed through the aluminium foil covering the second well. This well contains antibodies produced against the alpha-subunit of lutropin and to which biotin molecules have been chemically attached. The stick is kept in this well for 10 minutes, removed and pushed through aluminium foil covering the third well, containing a second rinsing solution. The stick is removed from the third well immediately and used to pierce the aluminium foil covering the fourth well. This well contains alkaline phosphatase enzyme, to which avidine molecules have been attached by chemical means. The stick is kept in this well for 10 minutes, removed and used to pierce the aluminium foil covering the fifth well. This well contains a third rinsing solution. The stick is withdrawn immediately and used to pierce the aluminium foil covering the sixth well. This well contains chromogenic substrate for alkaline phosphatase enzyme. The alkaline phosphatase removes the phosphate group from the chromogenic substrate molecule, as a result of which the molecules produced react together or with a third molecule to form a coloured compound insoluble in water, which precipitates onto the surface of the testing stick. The stick is kept in this well for 10 minutes, removed and examined visually for immediate estimation of the test result.

## Claims

1. A test kit for chemical and immunochemical analyses comprising
   a) an apparatus having one or more wells (7) made of an impervious material, being covered by an impervious foil and containing all reagents including rinsing solutions necessary for a given chemical or immunochemical reaction in liquid state; and
   b) a testing stick having a sharp point (1) for piercing the impervious foil covering the wells of said apparatus.

2. A test kit according to claim 1 in which the sharp reactive point (1) of the testing stick is produced from polystyrene, polyvinylcloride, nitrocellulose, glass or paper.

3. A test kit according to claim 1 or 2 in which the covering foil of the reagent wells contains aluminum.

4. A test kit according to any of claims 1 to 3 in which the sharp end of the testing stick (1) is coated with antibodies, antigens or a hapten.

5. A test kit according to any of claims 1 to 4 used for the investigation of body fluids.

## Revendications

1. Trousse d'essai pour des analyses chimiques et immunochimiques comprenant :

a) un appareil comprenant un ou plusieurs puits (7) faits en matière imperméable, recouverts d'un feuillet imperméable et contenant tous les réactifs, y compris des solutions de rinçage, nécessaires pour une réaction chimique ou immunochimique donnée à l'état liquide, et

b) une tigette d'essai ayant une pointe aiguë pour percer le feuillet imperméable recouvrant les puits de l'appareil.

2. Trousse d'essai suivant la revendication 1, dans laquelle la pointe aiguë réactive (1) de la tigette d'essai est en polystyrène, poly-(chlorure de vinyle), nitrocellulose, verre ou papier.

3. Trousse d'essai suivant la revendication 1 ou 2, dans laquelle le feuillet de recouvrement des puits à réactifs contient de l'aluminium.

4. Trousse d'essai suivant l'une quelconque des revendications 1 à 3, dans laquelle l'extrémité aiguë de la tigette d'essai (1) est revêtue d'anticorps, d'antigènes ou d'un haptène.

5. Trousse d'essai suivant l'une quelconque des revendications 1 à 4, utilisée pour procéder à l'examen de liquides du corps.

**Patentansprüche**

1. Testpackung für chemische und immunchemische Analysen umfassend

a) eine Vorrichtung mit einer oder mehreren aus einem undurchlässigen Material hergestellten Vertiefung(en) (7), die mit einer undurchlässigen Folie bedeckt ist (sind) und alle Reagenzien einschließlich der Lösungen zum Spülen enthält (enthalten), die für eine angegebene chemische oder immunchemische Reaktion in flüssiger Phase notwendig sind; und

b) ein Teststäbchen mit einer scharfen Stelle (1) zum Durchstoßen der undurchlässigen Folie, die die Vertiefungen der Vorrichtung bedeckt.

2. Testpackung nach Anspruch 1, bei der die scharfe reaktive Stelle (1) des Teststäbchens aus Polystyrol, Polyvinylchlorid, Nitrozellulose, Glas oder Papier hergestellt ist.

3. Testpackung nach einem der Ansprüche 1 oder 2, bei der die Reagenzienvertiefungen abdeckende Folie Aluminium enthält.

4. Testpackung nach einem der Ansprüche 1 bis 3, bei der das scharfe Ende des Teststäbchens (1) mit Antikörpern, Antigenen oder einem Hapten beschichtet ist.

5. Testpackung nach einem der Ansprüche 1 bis 4, die zur Untersuchung von Körperflüssigkeiten verwendet wird.

FIG. 1

FIG. 2

FIG. 3

FIG. 4

FIG. 5